(19) Europäisches Patentamt
European Patent Office
Office européen des brevets

(11) **EP 4 042 937 A1**

(12) **EUROPÄISCHE PATENTANMELDUNG**

(43) Veröffentlichungstag:
**17.08.2022 Patentblatt 2022/33**

(21) Anmeldenummer: **21156357.2**

(22) Anmeldetag: **10.02.2021**

(51) Internationale Patentklassifikation (IPC):
*A61B 5/0537* (2021.01)    *A61B 5/22* (2006.01)
*A61B 5/00* (2006.01)

(52) Gemeinsame Patentklassifikation (CPC):
**A61B 5/0537; A61B 5/225; A61B 5/6895**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Fresenius Medical Care Deutschland GmbH**
**61352 Bad Homburg (DE)**

(72) Erfinder: **Moissl, Ulrich**
**61184 Karben (DE)**

(74) Vertreter: **Bobbert & Partner**
**Patentanwälte PartmbB**
**Postfach 1252**
**85422 Erding (DE)**

(54) **MEDIZINISCHER ELEKTRODENTRAEGER, SYSTEM UND VERFAHREN**

(57) Die vorliegende Erfindung betrifft einen medizinischen Elektrodenträger (100, 100') mit wenigstens einer leitenden Oberfläche (102) zu ihrem Einsatz als Elektrode in einer Impedanzmessung oder als Teil hiervon. Ferner weist der medizinische Elektrodenträger (100, 100') eine Funktionseinheit auf, welche wenigstens einen Sensor, einen Signalempfänger, einen Signalgeber, eine Kommunikationsvorrichtung, Auswerteeinheit, Kraftmessvorrichtung, Trainingsvorrichtung und/oder eine Anzeigevorrichtung (204) aufweist. Außerdem weist der medizinische Elektrodenträger (100, 100') einen Leiter (108, 108') zur elektrischen Verbindung der leitenden Oberfläche (102) mit einer Basiseinheit (202) auf oder ist mit einem solchen Leiter ausgestaltet. Die Basiseinheit (202) ist ihrerseits konfiguriert, um entlang des Leiters (108,108') den medizinischen Elektrodenträger (100, 100') mit Strom zu versorgen und/oder Spannungsinformationen für eine Impedanzmessung oder -analyse mittels des Leiters (108,108') zu erhalten.

Fig. 3

**EP 4 042 937 A1**

**Beschreibung**

Die vorliegende Erfindung betrifft einen medizinischen Elektrodenträger gemäß Anspruch 1 und ein System gemäß Anspruch 11. Ferner betrifft sie das hierin offenbarte Verfahren bzw. Gegenstände gemäß jeweils der Oberbegriffe oder Gattungsbegriffe dieser Ansprüche.

Patienten, die Fluidbehandlungen, wie beispielsweise eine Hämodialyse, Hämofiltration, Hämodiafiltration oder Peritonealdialyse ("PD") erhalten, unterliegen gewöhnlich einer engmaschigen Kontrolle. Neben der Überwachung der jeweiligen Konzentrationen unterschiedlicher Blutbestandteile ist eine kontinuierliche Beobachtung des Hydrierungszustandes von Patienten entscheidend. Aus dem Stand der Technik ist der Einsatz der Impedanzanalyse zu diesem Zweck bekannt, bei welcher unter Verwendung von über Hautelektroden eingeleitetem Wechselstrom der Widerstand des Patientenkörpers bestimmt wird. Über die Bestimmung von Wirk- und Blindwiderstand lassen sich Rückschlüsse auf Fettmasse, und Muskelmasse des Patienten, aber auch auf dessen Körperwasser und dessen Hydrierungszustand ziehen.

Eine Aufgabe der vorliegenden Erfindung kann darin gesehen werden, einen zu seinem Einsatz in einem solchen Impedanzmessverfahren geeigneten medizinischen Elektrodenträger, ein System und ein Verfahren anzugeben.

Die erfindungsgemäße Aufgabe kann durch einen medizinischen Elektrodenträger mit den Merkmalen des Anspruchs 1 und durch ein System mit den Merkmalen des Anspruchs 11 gelöst werden.

Zudem kann sie durch ein hier offenbartes Verfahren gelöst werden.

Der erfindungsgemäße medizinische Elektrodenträger (im Folgenden auch kurz: Elektrodenträger) weist wenigstens eine leitende Oberfläche auf, welche zu ihrem Einsatz als Elektrode in einer Impedanz- oder bioelektrischen Impedanzmessung, oder als Teil einer solchen Elektrode, ausgestaltet ist.

Wenn hierin von einer "Impedanzmessung" die Rede ist, so kann dies eine bioelektrische Impedanzmessung (auch: Bioimpedanzmessung) sein. Analoges gilt hierin für den Begriff der "Impedanzanalyse".

Der medizinische Elektrodenträger weist ferner eine Funktionseinheit auf, welche ihrerseits vorzugsweise wenigstens ein Sensor, welcher nicht der Impedanzmessung oder der Messung von Widerstand, Strom oder Spannung auf oder über der Haut dient, ein Signalempfänger, ein Signalgeber, eine Kommunikationsvorrichtung, eine Auswerteeinheit, ein Tastkopf, eine Kraftmessvorrichtung, eine Trainingsvorrichtung, eine optische Einrichtung, z. B. Kamera, welche die Bestimmung der Lage des Elektrodenträgers ermöglicht, und/oder eine Anzeigevorrichtung, oder eine beliebige Kombination von zwei oder mehr der vorgenannten Elemente wie Sensoren, Signalempfänger, Signalgeber, usw., ist oder aufweist, wobei vorstehend genannte Elemente, gemeinsam oder unabhängig voneinander, vorzugweise jeweils nicht zur Impedanzmessung oder der Messung von Widerstand, Strom oder Spannung auf oder über der Haut dienen.

Weiter weist der medizinische Elektrodenträger einen elektrischen Leiter, oder ein Kabel, auf oder ist mit einem Anschluss für einen solchen Leiter ausgestaltet. Der Anschluss kann z. B. ein Verbinder, Stecker, eine Buchse und/oder dergleichen sein oder aufweisen.

Der Leiter, oder das Kabel, dient zur elektrischen Verbindung der leitenden Oberfläche mit einer weiteren Vorrichtung, beispielsweise einer Basiseinheit, welche ihrerseits zum Versorgen der leitenden Oberfläche mit Strom und/oder zum Auswerten von entlang des Leiters übermittelten Spannungsinformationen mittels Impedanzmessung oder -analyse konfiguriert ist.

Die weitere Vorrichtung, z. B. die Basiseinheit, ist nicht Teil des Elektrodenträgers. Der Elektrodenträger kann mit der Basiseinheit verbunden werden und ist es im Rahmen des erfindungsgemäßen Systems auch.

Das erfindungsgemäße System zum Bestimmen der Körperzusammensetzung eines Nutzers, welcher gleichzeitig Patient sein kann, mittels Impedanzanalyse weist wenigstens einen erfindungsgemäßen medizinischen Elektrodenträger und wenigstens eine Basiseinheit, zum Durchführen einer Impedanz- oder bioelektrischen Impedanzanalyse, auf.

Der medizinische Elektrodenträger und die Basiseinheit sind mittels eines Leiters zur Spannungsübertragung miteinander verbunden oder zur gemeinsamen Verbindung mittels des Leiters vorbereitet.

Die vorliegende Erfindung betrifft weiter ein Verfahren zum Verwenden des erfindungsgemäßen Systems und/oder des erfindungsgemäßen medizinischen Elektrodenträgers.

Das Verfahren umfasst das Bereitstellen eines erfindungsgemäßen Systems. Es betrifft ferner das Erheben von Daten, den Nutzer betreffend, mittels der Funktionseinheit des medizinischen Elektrodenträgers, welcher Teil des erfindungsgemäßen Systems sein kann. Das Verfahren kann ferner das Senden dieser Daten mittels der zugleich als Kommunikationsvorrichtung ausgestalteten und/oder programmierten Funktionseinheit umfassen.

Wann immer hierin eine Eignung oder ein Verfahrensschritt genannt ist, umfasst die vorliegende Erfindung vorzugsweise auch eine entsprechende Programmierung oder Konfigurierung einer geeigneten, insbesondere erfindungsgemäßen, Vorrichtung oder eines Abschnitts hiervon.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche und Ausführungsformen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Wenn hierin von "programmiert" oder "konfiguriert" die Rede ist, so ist auch offenbart, diese Begriffe gegeneinander auszutauschen.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen, und umgekehrt.

In einigen Ausführungsformen ist der medizinische Elektrodenträger oder dessen leitende Oberfläche ausgestaltet als wenigstens eine Handelektrode, als ein handgehaltenes Gerät und/oder als wenigstens eine Fußelektrode. Alternativ oder ergänzend weist er/sie eine Handelektrode oder eine Fußelektrode auf oder ist ein Teil hiervon.

Über den Leiter bzw. das Kabel kann in manchen Ausführungsformen sowohl die Energieversorgung des medizinischen Elektrodenträgers als auch der Datenaustausch zwischen Elektrodenträger, insbesondere Funktionseinheit, und Basiseinheit realisiert sein.

Hierin genannte Vorrichtungen und Eigenschaften der Funktionseinheit können in einigen Ausführungsformen ergänzend oder alternativ Vorrichtungen oder Eigenschaften der Basiseinheit sein.

In manchen Ausführungsformen weist die Basiseinheit einen Netzanschluss auf oder ist für einen solchen Anschluss vorbereitet.

Die Basiseinheit und/oder die Funktionseinheit können in einigen Ausführungsformen eine oder mehrere Speichervorrichtungen, beispielsweise zum Hinterlegen einer Datenbank, umfassen oder hiermit verbunden sein. Die Speichervorrichtung kann geeignet und/oder vorgesehen sein, Behandlungsdaten des Nutzers, Referenzdaten eines Kollektivs und/oder dergleichen zu speichern bzw. vorzuhalten und/oder dergleichen tatsächlich speichern bzw. vorhalten.

In manchen Ausführungsformen ist die Funktionseinheit des medizinischen Elektrodenträgers als Kommunikationsvorrichtung zum Senden und/oder Empfangen von Daten ausgestaltet und/oder programmiert. Die Daten können in exemplarischen Ausführungsformen mittels der Funktionseinheit gemessen worden sein, z. B. mittels eines Sensors, den die Funktionseinheit ebenfalls umfassen kann. Alternativ oder ergänzend können Daten der Funktionseinheit z. B. vom Nutzer zur Verfügung gestellt worden sein, z. B. Angaben zu Besonderheiten im Tagesablauf (z. B. Nutzung des medizinischen Elektrodenträgers im nüchternen oder im postprandialen Zustand), Angaben zum Befinden, personenbezogene Daten (Name, Gewicht, usw.) und weitere. Auch diese Daten können, wie vorstehend ausgeführt und nachstehend detaillierter erläutert, gesendet werden, z. B. an die Basiseinheit.

In einigen Ausführungsformen ist die Funktionseinheit des Elektrodenträgers als Kommunikationsvorrichtung eingerichtet, um Daten an wenigstens eine weitere Vorrichtung zu senden und/oder um Daten von wenigstens einer weiteren Vorrichtung zu empfangen.

Die weitere Vorrichtung kann ihrerseits vorzugsweise jeweils eine eigene, leitende Oberfläche zu ihrem Einsatz als Elektrode in einer Impedanzmessung aufweisen. Alternativ oder ergänzend ist die weitere Vorrichtung als weiterer erfindungsgemäßer medizinischer Elektrodenträger wie hierin offenbart ausgestaltet.

Damit kann in solchen Ausführungsformen ein Elektrodenträger vorliegen, der konfiguriert ist, um mit einem zweiten, von ihm unabhängig bewegbaren Elektrodenträger Daten auszutauschen.

In manchen Ausführungsformen ist die Funktionseinheit als Kommunikationsvorrichtung zum Senden von Daten an und/oder zum Empfangen von Daten von der Basiseinheit ausgestaltet und/oder programmiert. Die Daten sind hierbei vorzugsweise keine Spannungsinformationen, etwa im Sinne der Impedanzmessung oder -analyse, oder zu deren Nutzung, z. B. in einer Grundform der Impedanzmessung oder -analyse, oder nicht nur solche Daten, sondern sind oder umfassen z. B. Vitaldaten des Nutzers, Ergebnisse einer Kraftmessung, usw.

In einigen Ausführungsformen des medizinischen Elektrodenträgers ist dessen Funktionseinheit, wenn sie als Kommunikationsvorrichtung dient, als Radarsender- und/oder Radarempfänger ausgestaltet oder umfasst dergleichen.

Ein Radarsender, wie hierin verwendet, ist eine Sendevorrichtung für Radarwellen. Er ist beispielsweise eine Vorrichtung, welche ein sogenanntes Primärsignal als gebündelte elektromagnetische Welle aussendet. Ein Radarempfänger, wie hierin verwendet, ist eine Empfangsvorrichtung für Radarwellen. Er ist beispielsweise eine Vorrichtung, welche von Objekten reflektierte Echos oder bei Transmission das abgeschwächte Primärsignal als Sekundärsignal empfängt. Das Sekundärsignal kann nach vorbestimmten Kriterien ausgewertet werden.

Eine Radarwelle kann eine elektromagnetische Welle, d. h. eine Welle aus gekoppelten elektrischen und magnetischen Feldern, sein. Beispiele für elektromagnetische Wellen sind Radiowellen, Mikrowellen, Wärmestrahlung, Licht, Röntgenstrahlung und Gammastrahlung. Elektromagnetische Wellen im Vakuum sind Transversalwellen. Die Wechselwirkung elektromagnetischer Wellen mit Materie hängt von ihrer Frequenz ab.

Der Radarsender kann ausgestaltet sein, um Radarwellen mit einer Frequenz f von 1 bis 300 GHz, was einer Wellenlänge von ca. 30 cm bis 1 mm entspricht, auszusenden.

Radarwellen können (Nahfeld-)Radarwellen sein.

Die gesendeten Daten sind oder umfassen in diesen Ausführungsformen vorzugweise Lagedaten des Elektrodenträgers, z. B. im Raum, oder Abstandsdaten, etwa Daten, die den Abstand des Elektrodenträgers zu einer weiteren Vorrichtung oder Struktur angeben oder diesen aus ihnen errechnen lassen. Die weitere Vorrichtung oder Struktur kann ein weiterer Elektrodenträger, z. B. wie hierin offenbart, sein.

In manchen Ausführungsformen ist die Kommunikationsvorrichtung eine Vorrichtung zum Verbinden mit einem WLAN (Wireless Local Area Network), einem Mobilfunknetz, Bluetooth, NFC (Near Field Communication), Radar, 3G, LTE, 4G, 5G, ZigBee, NFC, Wibree und/oder WiMAX und/oder dergleichen oder umfasst eine solche.

In bestimmten Ausführungsformen kann zusätzlich mithilfe der Kommunikationsvorrichtung eine Verbindung zu einem lokalen Netzwerk und/oder einem Wide Area Network hergestellt werden, um Daten mit einem Remote Server oder einer Datencloud auszutauschen. Eine entsprechende Vorbereitung, Programmierung, usw. kann vorgesehen sein. Unter Daten sind hierbei, wie auch an jeder anderen Stelle der vorliegenden Offenbarung, vorzugsweise sämtliche oder beliebige Daten zu verstehen, die der erfindungsgemäße medizinische Elektrodenträger und/oder eine Basiseinheit messen und/oder bestimmen können.

In einigen Ausführungsformen weist die Funktionseinheit des medizinischen Elektrodenträgers eine Anzeigevorrichtung auf. Diese kann programmiert oder konfiguriert sein zum Anzeigen von Daten, welche vom medizinischen Elektrodenträger oder dessen Funktionseinheit erhoben oder ihm/ihr übermittelt wurden. Die Anzeigevorrichtung kann dem Anzeigen der Daten zur Kenntnisnahme durch einen Nutzer dienen.

In manchen Ausführungsformen weist der erfindungsgemäße medizinische Elektrodenträger wenigstens oder genau eine weitere, leitende Oberfläche, insbesondere wie hierin beschrieben, auf. Somit sind wenigstens zwei leitende Oberflächen, vorzugsweise getrennt voneinander und/oder an unterschiedlichen Positionen auf oder an einem medizinischen Elektrodenträger angeordnet.

In einigen Ausführungsformen weisen die Funktionseinheit oder die Basiseinheit wenigstens einen Sensor und/oder eine Ausgabevorrichtung zum Bestimmen und Ausgeben eines Werts, welcher der aktuellen Lebensqualität des Nutzers entspricht oder dieser zugeschrieben ist, auf.

Die Ausgabevorrichtung kann konfiguriert sein zum Bestimmen und Ausgeben eines Werts basierend auf von ihr mittels Sensoren erhobenen Daten, denen - einzeln oder in Kombination und/oder Wechselwirkung miteinander - ein Wert auf einer Lebensqualitätsskala zugewiesen werden kann. Der Wert kann basierend auf den erhobenen Daten unter Rückgriff auf gespeicherte Vergleichswerte desselben Nutzers oder eines Kollektivs von Nutzern ermittelt werden, etwa mittels einer Nachschlagetabelle, eines Datenspeichers mit einer Vielzahl von gespeicherten Werten, usw. Der auf diese oder andere Weise erhobene oder ermittelte Wert kann als die vom Nutzer empfundene oder für diesen objektiv angenommene Lebensqualität, als Zahlenwert, als quantitativer Wert, als qualitative Aussage usw. ausgegeben werden. Hierzu kann auf Listen, Tabellen, Zuordnungen und dergleichen zurückgegriffen werden, in welchen vorab festgehalten wurde, welcher Handkraft oder -veränderung das Ergebnis einer subjektiven Bewertung oder objektiven Messung zugeordnet wurde.

In manchen Ausführungsformen weist die Funktionseinheit einen Sensor zum Bestimmen der Handkraft des Nutzers auf. Dieser Sensor kann eine Kraftmessvorrichtung oder Teil einer Kraftmessvorrichtung sein.

In einigen Ausführungsformen des erfindungsgemäßen Systems ist die Basiseinheit oder eine Recheneinrichtung des Systems, welche Teil der Basiseinheit sein kann, programmiert, um mittels Impedanzanalyse einen Wert für die Muskelmasse oder für das Lean tissue des Nutzers zu berechnen. Ferner ist die Basiseinheit oder Recheneinrichtung in diesen Ausführungsformen programmiert, basierend sowohl auf dem mittels Impedanzanalyse berechneten oder gemessenen Wert $LT_{measure}$ als auch auf der bestimmten Handkraft des Nutzers einen korrigierten Wert für die Muskelmasse oder für das Lean tissue des Nutzers zu berechnen. Dies kann der Optimierung des ermittelten Werts für die Muskelmasse oder für das Lean tissue dienen.

Fresenius Medical Care Deutschland GmbH

Das Berechnen des korrigierten Werts anhand oder unter Berücksichtigung der gemessenen Handkraft kann z. B. durch (lineare) Regression, AI, auf Basis eines Datensatzes, Addition, Subtraktion, Mittelung mit oder ohne Gewichtung, Berücksichtigung eines Faktors und/oder dergleichen erfolgen.

In manchen Ausführungsformen ist von der vorliegenden Erfindung umfasst, die gemessene Handkraft mit einer früher ermittelten Muskelmasse (oder Lean tissue) des Patienten und/oder mit vorab festgelegten Sollwerten in Relation zu setzen, insbesondere mittels Diagrammen, Graphiken und/oder dergleichen. Ausgehend von dieser Relation erhält man für eine bestimmte Handkraft (z. B. in [N]) einen, dieser Handkraft zugeordneten, Wert für die Muskelmasse (z. B. in [kg]), etwa durch Nachschlagen, Ablesen, usw. Dieser Wert kann zur Korrektur des mittels Impedanzanalyse erhaltenen Werts für die Muskelmasse genutzt werden.

In einigen Ausführungsformen kann zum Ermitteln des korrigierten Werts $LT_{correct}$ für die Muskelmasse des Patienten, der Ergebniswert der Impedanzanalyse und der Wert $LT_{handgrip}$, welcher der bestimmten Handkraft zugeordnet ist, unterschiedlich gewichtet werden oder eine solche Gewichtung umfassen.

So kann der korrigierte Werts $LT_{correct}$ beispielsweise als

$$LT_{correct} = w1 \times LT_{measure} + w2 \times LT_{handgrip} \qquad (1)$$

berechnet werden und damit einem gewichteten Mittelwert mit den (exemplarischen) Gewichten **w1 = 80 %** und **w2 = 20 %** entsprechen, wobei Fresenius Medical Care Deutschland GmbH

$LT_{correct}$     der korrigierte Wert des Lean tissue
$LT_{measure}$     der Wert des Lean tissue basierend auf der Impedanzanalyse und
$LT_{handgrip}$     der Wert des Lean tissue basierend auf der mittels Messung bestimmten Handkraft und entsprechender Zuordnung von Handkraft zu Muskelmasse,

ist. Der Wert $LT_{handgrip}$ der Muskelmasse/des Lean tissue (beide Begriffe sind hierin gleichbedeutend verwendet) basierend auf der bestimmten Handkraft kann, wie vorstehend ausgeführt, beispielsweise aus den oben genannten Relationen, insbesondere aus Graphiken, ermittelt werden.

So könnte rein exemplarisch die Messung eines Patienten mittels BCM den folgenden Wert ergeben:

$$LT_{measure} = 53\ kg,$$

und die Messung der Handkraft, wenn in Relation mit dem Lean tissue gesetzt, beispielsweise

$$LT_{handgrip} = 51\ kg.$$

Eingesetzt in Gleichung (1) ergibt sich ein korrigierter Wert $LT_{correct}$ für das Lean tissue von

$$LT_{correct} = 80\ \% \times 53\ kg + 20\ \% \times 51\ kg = 52{,}6\ kg$$

Dieses Ergebnis, welches basierend auf einer gewichteten Mittelwertbildung von Werten, mittels zweier unabhängiger Methoden gewonnen wurde, ist genauer als die Ergebnisse der Fresenius Medical Care Deutschland GmbH einzelnen Methoden, vor allem sofern die Varianzen in etwa bekannt sind.

In einigen Ausführungsformen können die Kraftmessvorrichtung oder der Sensor zum Bestimmen der Handkraft des Nutzers die leitende Oberfläche und/oder die weitere leitende Oberfläche aufweisen.

In manchen Ausführungsformen können die Funktionseinheit oder die Basiseinheit ausgestaltet sein, um den Einfluss der Hautoberfläche bei der Analyse der Impedanz rechnerisch zu extrahieren, z. B. indem sie Daten liefern, aufgrund welcher der Einfluss der Hautoberfläche bei der Analyse der Impedanz berücksichtigt werden kann, etwa bei ihrem Eingang in Berechnungen bei der Impedanzanalyse.

In bestimmten Ausführungsformen können die bei einer Kraftmessung erhobenen Daten, die Vitaldaten des Patienten, Daten betreffend des Fluidstatus des Patienten und/oder Untersuchungsdaten, die unter Verwendung einer Vorrichtung generiert worden sind, und weitere Daten beispielsweise zur weiteren Analyse und/oder zu Archivierungszwecken über das Datennetzwerk, wie hierin beschrieben, ausgetauscht werden. Die hierin genannten Vorrichtungen können entsprechend konfiguriert sein.

In einigen Ausführungsformen ist die Funktionseinheit als Trainingsvorrichtung zum Trainieren der Handmuskulatur des Nutzers ausgestaltet und/oder programmiert.

In manchen Ausführungsformen kann die Trainingsvorrichtung einen Energiespeicher, eine Feder, einen Sensor und/oder eine hierzu geeignete Mechanik aufweisen.

Fresenius Medical Care Deutschland GmbH

Beispielsweise können hiermit im Falle einer erkannten Kraftabnahme des Nutzers, vor allem wenn die Kraft nachhaltig, kontinuierlich oder stetig, beispielsweise über Messungen, die an zwei, drei oder mehr Tagen stattfinden, abnimmt, zusätzliche Trainingseinheiten mit der Kraftmessvorrichtung vorgeschlagen und/oder vorgeschrieben werden. Dies kann entweder automatisch durch das erfindungsgemäße System initiiert und/oder von einer externen Instanz bestimmt werden. Die externe Instanz kann ein externes Computersystem sein, das mit entsprechender Erkennungssoftware ausgestattet ist, medizinisches Personal, welches eine Trainingsverschreibung an das System senden kann, oder dergleichen.

Von der vorliegenden Erfindung ist weiter umfasst, dass zur Steigerung der Motivation des Nutzers, Kraftübungen mittels des Elektrodenträgers zu machen, "Gamification"-Elemente Einsatz finden. So können beispielsweise Nutzer vergleichbaren Alters und körperlicher Verfassung ihre Trainingsleistung miteinander vergleichen, indem eine entsprechende Rangliste, beispielsweise auf einem Display des medizinischen Elektrodenträgers, der Basiseinheit oder an anderer Stelle des erfindungsgemäßen Systems, welches das Smartphone, ein Tablet-Computer oder dergleichen umfassen kann, bereitgestellt wird.

Weiter ist von der vorliegenden Erfindung umfasst, dass sich Nutzer direkt miteinander verbinden oder in Kontakt miteinander treten können, z. B. mittels des verwendeten Elektrodenträgers oder Systems, um gegeneinander in einem Wettbewerb anzutreten.

Fresenius Medical Care Deutschland GmbH

Alternativ oder ergänzend kann eine visuelle und/oder akustische Meldung nach dem Training generiert werden, die eine relative Einordnung der Trainingsleistung bietet, z. B. "Sie sind 78 % stärker als vergleichbare Peritonealdialyse("PD")-Patienten in Ihrer Altersklasse".

In bestimmten Ausführungsformen sind das Ermitteln und/oder Ausgeben oder Anzeigen von virtuellen "Achievements" von der vorliegenden Erfindung umfasst, die nach dem Erreichen eines Trainingsziels dem Nutzer und/oder Dritten, z. B. medizinischen Fachkräften, Freunden und/oder Familienangehörigen, angezeigt werden, z. B. um die Langzeitmotivation des Nutzers zu steigern.

Um den Trainingszustand, die Differenz zum Trainingsziel oder weitere Informationen dem Nutzer und/oder Dritten, z. B. medizinischen Fachkräften, Freunden und/oder Familienangehörigen, anzeigen zu können, kann vorgesehen sein, mittels der Funktionseinheit oder mittels der Basiseinheit vorstehende oder andere Informationen auf eines oder mehrere mobile Endgeräte (wie Smartphone, Tablet, Notebook, usw.) zu übertragen, welche Teil des erfindungsgemäßen Systems sein können und/oder mit der Funktionseinheit oder Basiseinheit in Signalverbindung stehen oder hierzu vorbereitet sein können.

Hinweise auf erzielte Leistungen können z. B. an der Basiseinheit und/oder dem Elektrodenträger oder beliebigen anderen Komponenten des erfindungsgemäßen Systems, wie insbesondere vorstehend genannte mobile Endgeräte, optisch, haptisch oder akustisch codiert sein, beispielsweise als Darstellungen auf einer Anzeigevorrichtung, Vibrieren, als Ton oder als vorbestimmte Melodie.

Fresenius Medical Care Deutschland GmbH

Mögliche Hinweise, und vor allem optische Darstellungen können in Form von Diagrammen (z. B., Skala, Angabe des Perzentils), Rankings mit Platzierung gegen andere User wie Freunde, Bekannte sein. Hinweise auf Achievements können beispielsweise mittels Graphiken (Piktogrammen wie Sternchen, Pokale, Emojis, usw.) einhergehen.

In manchen Ausführungsformen bestehen Signalverbindung oder -kommunikation oder die erforderlichen Vorbereitung für eine Signal- oder Kommunikationsverbindung zwischen hierin genannten Komponenten des Systems oder des Elektrodenträgers.

Wenn hierin von einer Signal- oder Kommunikationsverbindung zweier Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebundenen, kabellosen oder auf andere Weise umgesetzten) Signalkommunikation besteht, beispielsweise durch eine Kopplung beider Komponenten, etwa mittels *pairing,* usw.

Unter *pairing* versteht man einen Prozess, der im Zusammenhang mit Rechnernetzwerken erfolgt, um eine anfängliche Verknüpfung zwischen Rechnereinheiten zum Zwecke der Kommunikation herzustellen. Das bekannteste Beispiel hierfür ist das Herstellen einer Bluetooth-Verbindung, mittels welcher verschiedene Einrichtungen (z. B. Smartphone, Kopfhörer) miteinander verbunden werden. *Pairing* wird gelegentlich auch als *bonding* bezeichnet.

In einigen Ausführungsformen ist die Funktionseinheit wenigstens ein Sensor zum Bestimmen eines oder mehrerer Fresenius Medical Care Deutschland GmbH Vitalparameter des Nutzers oder weist einen solchen Sensor auf.

In bestimmten Ausführungsformen können solche Sensoren zum Erfassen von Herzschlag, Glukosespiegel und/oder Sauerstoffsättigung konfiguriert und vorgesehen sein.

In manchen Ausführungsformen können Sensoren zu vorstehendem oder zu anderem Zweck als Grünlichtsensoren ausgestaltet sein, beispielsweise wie in der US 5,830,137 A der University of South Florida offenbart, deren diesbezügliche Offenbarung hiermit per Verweis auch zum Gegenstand der vorliegenden Offenbarung gemacht wird. Zusätzlich oder alternativ können auch Radarsensoren für die Bestimmung von Herzfrequenz und Atmung und/oder klassische Pulsoximeter, z. B. mittels Rotlicht, zur Messung der Sauerstoffsättigung vorgesehen sein.

Ebenfalls von der Erfindung umfasst ist, dass ein oder mehrere Sensoren die Handtemperatur des Patienten messen, um so beispielsweise Fieber zu detektieren und ggf. anzuzeigen. Gängige Technologien zur Temperaturbestimmung sind z. B. NTC-Thermistoren und/oder Infrarotsensoren.

Alternativ oder zusätzlich ist von der Erfindung umfasst, dass mittels zweier leitenden Oberflächen, die insbesondere als Leitfähigkeitssensoren ausgestaltet und vorzugsweise an einem gemeinsamen Elektrodenträger angeordnet sind, die Leitfähigkeit der Haut gemessen und somit die Hautfeuchtigkeit ermittelt werden kann.

Eine erhöhte Feuchtigkeit kann beispielsweise ein Hinweis auf Fieber sein. Dies kann in manchen Fällen auf zu warme(s) Fresenius Medical Care Deutschland GmbH Dialysierflüssigkeit, Substituat, oder anderes eingesetztes Fluid (z. B. für die Peritonealbehandlung) zurückzuführen sein, so dass eine Kontrolle der Temperatur des Fluids vorgeschlagen werden könnte.

Die gemessene Feuchtigkeit kann alternativ oder ergänzend zur besseren Einschätzung des Hautübergangswiderstands oder des Widerstands zwischen Körper und Elektrode verwendet werden, welche bei der Bioimpedanzanalyse Berücksichtigung finden könnte.

Werden Klebeelektroden verwendet, kann die Ermittlung der Hautfeuchtigkeit herangezogen werden, um deren adhäsive Eigenschaften besser einschätzen zu können, die sich ebenfalls auf die Messung auswirken können. Beispielsweise könnte ein bestimmter Wert der Handfeuchtigkeit oberhalb eines Grenzwertes eine Benachrichtigung an den Nutzer veranlassen, welche eine Aufforderung zur Überprüfung des Sitzes der Klebeelektroden nach sich ziehen kann.

Da eine erhöhte Handfeuchtigkeit auf eine erhöhte Körpertemperatur und somit auf eine erhöhte Durchblutung zurückzuführen sein könnte, könnte dies eine Überschätzung der Impedanz und somit eine Überschätzung der Überwässerung nahe legen. In diesen Fällen kann die Ermittlung eines Korrekturwerts mit der Messung der Handfeuchtigkeit einhergehen.

Eine regelmäßige Messung der Vitalparameter erlaubt eine detailliertere Überwachung von Patienten beispielsweise vor/während/nach einer Behandlung, einer Impedanzmessung, und/oder einer Handkraftmessung. Anhand der gemessenen Daten lässt sich ein genaueres Bild des Fitnesszustandes und/oder Fresenius Medical Care Deutschland GmbH des Gesundheitszustandes des Nutzers ableiten. Hierdurch können bereits frühzeitig Gegenmaßnahmen getroffen werden, falls ein für den Patienten ungünstiger Fitness- und/oder Gesundheitszustand gemessen und/oder vorhergesagt wird. Wird beispielsweise vor einer Fluidbehandlung festgestellt, dass der Patient gegenwärtig unter Kreislaufproblemen leidet, kann z. B. die Dialysedosis angepasst und/oder dem Nutzer eine Nahrungsaufnahme empfohlen werden, um einer weiteren Verschlechterung seines Zustandes entgegenzuwirken.

Weiterhin ist von der vorliegenden Erfindung umfasst, dass Vitalparameter, wie beispielsweise Herzfrequenz und/oder Atemfrequenz, nach einer Handkraftübung gemessen werden, um z. B. frühzeitig eine Herz- und/oder Kreislaufschwäche erkennen zu können. Eine entsprechende Aufzeichnung, Speicherung und/oder Ausgabe kann vorgesehen sein.

In einigen Ausführungsformen weist die Funktionseinheit wenigstens einen Sensor zum Bestimmen der Hydrierung des Nutzers oder eines seiner Körperteile auf.

In manchen Ausführungsformen ist die Funktionseinheit ein Ultraschalltastkopf oder weist einen solchen auf.

Beispielsweise kann mit dem Ultraschalltastkopf die Lunge oder der von der Pleura umfasste Raum sonografisch untersucht werden, um Überwässerungen des Nutzers zu ermitteln, indem Flüssigkeitsansammlungen innerhalb der Lunge oder der Pleura detektiert werden, welche besonders bei ESRD (end-stage renal disease) Patienten auftreten. Integrierte Ultraschalltastköpfe bieten somit den Vorteil einer regelmäßig zugänglichen Überwachung der Lunge, auch um beispielsweise einen Pneumothorax frühzeitig zu erkennen.

Fresenius Medical Care Deutschland GmbH

In einigen Ausführungsformen ist die Funktionseinheit wenigstens eine Auswerteeinheit für die von ihr gemessenen Daten und/oder zum Ausgeben einer Diagnose, etwa in Verbindung mit einem Datenspeicher, oder sie umfasst eine solche.

In bestimmten Ausführungsformen kann die Auswerteeinheit der Ortsbestimmung des medizinischen Elektrodenträgers dienen, z. B. als Absolutmessung, und/oder der Lagebestimmung des medizinischen Elektrodenträgers relativ zu weiteren Vorrichtungen.

In manchen Ausführungsformen des erfindungsgemäßen Systems weist die Basiseinheit wenigstens eine Schnittstelle zum Anschließen einer oder mehrerer Elektroden mit leitenden Oberflächen und/oder eines oder mehrerer medizinischen Elektrodenträger(s), wie hierin offenbart, auf.

In einigen Ausführungsformen ist oder umfasst das System ein berührungsempfindliches Display. Das Display ist vorzugsweise konfiguriert oder programmiert zum Steuern oder Bedienen des Systems oder der Basiseinheit durch den Nutzer.

In manchen Ausführungsformen des Systems weisen der medizinische Elektrodenträger und/oder die Basiseinheit akustische, optische oder elektromagnetische, insbesondere gestengesteuerte, Kommunikationseinrichtungen auf. Diese dienen zur Kommunikation mit einem Nutzer und/oder zu ihrer Bedienung durch den Nutzer, oder zur Kommunikation untereinander.

Fresenius Medical Care Deutschland GmbH

Typische Beispiele für Kommunikationseinrichtungen, die einen Datenaustausch auf Basis elektromagnetischer Trägersignale erlauben, sind beispielsweise Radar, Bluetooth, WLAN, 3G, LTE, 4G, 5G, ZigBee, NFC, Wibree und/oder WiMAX. Durch die Verwendung elektromagnetischer Trägersignale, welche durch die in dem medizinischen Elektrodenträger implementierten Kommunikationseinrichtungen abgegeben werden, ist es beispielsweise möglich, den Abstand und/oder die genaue Position weiterer Vorrichtungen und/oder der Basiseinheit relativ zueinander mit guter Genauigkeit, z. B. mithilfe einer time-of-flight Messung, zu bestimmen.

Der resultierende technische Effekt kann eine Gewährleistung der korrekten Handhabung des erfindungsgemäßen medizinischen Elektrodenträgers bzw. des erfindungsgemäßen Systems während/vor/nach einer Impedanzmessung oder deren korrekten Auswertung begünstigen. Letzteres kann insbesondere dann von Vorteil sein, wenn Nutzer im heimischen Umfeld behandelt werden und sie die Impedanzmessung ohne Hilfe von Fachkundigen durchführen müssen.

Ebenso lässt sich mithilfe entsprechender Kommunikationsvorrichtungen zum Datenaustausch, insbesondere mittels geeigneter Sensoren und/oder mittels der Radarsender/-empfänger, die Genauigkeit der Impedanzmessung verbessern, da die Messposition, z. B. Arme seitlich/vorne am Körper angelegt, Arme seitlich/vor dem Körper ausgestreckt, sowie der Abstand der medizinischen Elektrodenträger voneinander, entscheidend in die Bestimmung des Fluidstatus oder des Hydrierungsstatus des Nutzers miteinfließen können.

In manchen Ausführungsformen ist umfasst, dass das System dem Nutzer unterschiedliche Messpositionen vorschlägt und/oder Fresenius Medical Care Deutschland GmbH entsprechende Anweisungen ausgibt, z. B. durch eine Textnachricht, ein Piktogramm und/oder eine Sprachnachricht, damit er/sie eine für die Messung vorteilhafte Haltung einnehmen kann. Die Haltung kann, z. B. über die Kommunikationsvorrichtungen der medizinischen Elektrodenträger, beispielsweise durch Ermittlung des Abstands zwischen diesen, während der gesamten Messdauer verfolgt werden, sodass Fehlanwendungen vermieden werden, was zu einer höheren Genauigkeit der Impedanzmessung und einer präziseren Dialysedosisbestimmung führt.

Es kann hierzu in bestimmten Ausführungsformen ergänzend eine Benachrichtigung des Nutzers, insbesondere zur Korrektur seiner Körperhaltung, vorgesehen sein.

In einigen Ausführungsformen des Systems ist die Basiseinheit zum Bestimmen der Hydrierung des Nutzers oder eines seiner Körperteile und/oder zum Ausgeben eines entsprechenden Ergebnisses oder Werts ausgestaltet und/oder programmiert, oder weist eine Vorrichtung hierfür auf, insbesondere als Impedanzmessvorrichtung.

Aus dem Stand der Technik (z. B. WO 2010/022933 A1, deren diesbezügliche Offenbarung hiermit per Verweis zum Gegenstand auch dieser Offenbarung gemacht wird) sind Tastköpfe bekannt. Sie können erfindungsgemäß an den medizinischen Elektrodenträgern, insbesondere an deren Enden, angeordnet sein. Solche Tastköpfe ermöglichen es, eine, vorzugsweise lokale, Impedanz zu messen. Eine Ausführungsform mit solchen oder vergleichbaren Tastköpfen bietet die Möglichkeit, das Füllvolumen des Peritoneums mittels Dialyseflüssigkeit zu überwachen, indem die Tastköpfe der Elektroden rechts und Fresenius Medical Care Deutschland GmbH links an die Bauchwand gehalten werden, was hierin auch als lokale Impedanzmessung bezeichnet wird.

Das Durchführen solcher lokalen Impedanzmessungen kann von Vorteil bei der Bestimmung viszeraler Fettmasse sein. So kann bei kritischen Werten (zu viel/zu wenig Fettmasse) dem Nutzer z. B. eine entsprechende Ernährungsumstellung vorgeschlagen und/oder ein entsprechendes Fitnessprogramm eingerichtet werden.

In manchen Ausführungsformen umfasst die Basiseinheit wenigstens eine Auswerteeinheit zum Ausgeben einer Diagnose.

In manchen Ausführungsformen umfasst das Verfahren wenigstens einen der folgenden Schritte oder mehrere der folgenden Schritte in beliebiger Kombination:

- Messen der Distanz zwischen zwei Elektrodenträgern, insbesondere, wenn diese jeweils an die Bauchwand gehalten werden;

- Durchführen von Kraftübungen zum Bestimmen oder Messen der Kraft, mit welcher die Kraftmessvorrichtung betätigt wird;

- Ansetzen wenigstens eines Ultraschalltastkopfs des Elektrodenträgers auf die Haut des Nutzers zum Erzeugen von sonografischen Informationen, insbesondere Bildern,; und/oder

- Halten wenigstens einer der leitenden Oberfläche an die Bauchdecke zum Messen der lokale Impedanz und/oder des Füllvolumens des Peritoneums oder der Bauchhöhle.

Fresenius Medical Care Deutschland GmbH

Manche oder alle erfindungsgemäßen Ausführungsformen können einen, mehrere oder alle der oben und/oder im Folgenden genannten Vorteile aufweisen.

Im klinischen Umfeld werden in der Regel zur genauen Bestimmung des extra- bzw. intrazellulären Wassers mittels Impedanzanalyse Vorrichtungen wie beispielsweise der "Body Composition Monitor" ("BCM") der Anmelderin verwendet. Das Anlegen der Elektroden für die Impedanzanalyse und die Überwachung der Impedanzmessung übernimmt dabei üblicherweise medizinisch geschultes Personal, so dass Fehlanwendungen reduziert werden und der Fluidstatus des Patienten mit guter Zuverlässigkeit angegeben werden kann. Letzteres ist von großer Bedeutung, da unpräzise oder grob fehlerhafte Messungen zu falschen Einstellungen an der medizinischen Behandlungsvorrichtung führen können, mit der die Behandlung durchgeführt wird, was wiederum eine Gefahr für den Patienten darstellen kann.

Im Gegensatz zu Patienten, die ihre Fluidbehandlung in Kliniken oder Dialysezentren erhalten, unterliegen jene, die ihre Behandlung im heimischen Umfeld durchführen, beispielsweise im Rahmen einer Peritonealdialyse, einer deutlich grobmaschigeren Überwachung. Zudem sind diese Patienten bei der Bestimmung ihres Fluidstatus zumeist weitestgehend auf sich gestellt oder müssen zusätzliche Reisezeiten zu einer Klinik in Kauf nehmen, um eine adäquate Betreuung zu erhalten.

Die vorliegende Erfindung kann hier vorteilhaft abhelfen, indem sie dazu beiträgt, dass die Zusammensetzung des Körpers des Patienten, insbesondere dessen Fluidzustand, auch durch Fresenius Medical Care Deutschland GmbH den Nutzer der erfindungsgemäßen Vorrichtungen selber und ausreichend zuverlässig bestimmt werden kann. Medizinisches Personal ist hierfür nicht zwingend erforderlich, was überdies beitragen kann, Kosten einzusparen.

Da sich die vorliegende Erfindung zur regelmäßigen Verwendung durch den Patienten anbietet, ist weiter eine kontinuierliche Beobachtung des Fluidzustands des Körpers vorteilhaft möglich.

Ein weiterer Vorteil der vorliegenden Erfindung kann in dem sich hieraus ergebenden Beitrag zu einer verbesserten, weil engmaschigeren, Patientenüberwachung liegen. Dies trägt mittelbar zur Steigerung der Patientensicherheit bei.

Mittels der vorliegenden Erfindung kann weiter ein unerwünschter Einfluss der Hautoberfläche auf das Ergebnis der Impedanzanalyse extrahiert und damit die Präzision des Messverfahrens vorteilhaft verbessert werden.

Durch eine frühzeitige Erkennung eines reduzierten Fitnesszustands mittels der vorliegenden Erfindung kann rechtzeitig beispielsweise eine beginnende Sarkopenie erkannt werden, was zumeist mit einer Abnahme der Lebensqualität des Nutzers korreliert ist. So ist es vorteilhaft bereits frühzeitig möglich, durch Erkennung der Kraftabnahme eines Nutzers, eine gesundheitliche Verschlechterung sowie eine damit einhergehende Minderung der Lebensqualität zu erkennen oder gar zu prognostizieren und entsprechende Gegenmaßnahmen einzuleiten. Möglich ist die Eingabe des Gewichtes, z. B. mittels Interface oder via Cloud-Abruf von einer externen Datenbank. Gewichtsangaben können ebenfalls bei der Bewertung der Lebensqualität berücksichtigt sein.

Fresenius Medical Care Deutschland GmbH

Das frühzeitige Ermitteln der Lebensqualität und das hierdurch mögliche frühzeitige Gegensteuern durch Vorschlagen entsprechender Maßnahmen, können wiederum lebensverlängernd sein. Aus zahlreichen Studien ist bekannt, dass eine verminderte Lebensqualität mit einer signifikant erhöhten Mortalität einhergeht.

Obgleich hierin an verschiedenen Stellen Bezug auf die Dialyse, und insbesondere auf die Peritonealdialyse, genommen wird, sind die erfindungsgemäßen Gegenstände und ihre Verwendung nicht auf den Bereich der Dialyse oder Nutzer mit eingeschränkter Nierenfunktion beschränkt. Die vorliegende Erfindung kann vielmehr ergänzend bei Patienten beliebiger Erkrankung, insbesondere geriatrischen Patienten oder Krebspatienten, oder bei gesunden Nutzern im Zuge der Ernährungsüberprüfung usw. vorteilhaft eingesetzt werden.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:

**Fig. 1**   zeigt eine exemplarische Ausführungsform des erfindungsgemäßen medizinischen Elektrodenträgers, hier als Handelektrode;

**Fig. 2**   zeigt eine erste exemplarische Ausführungsform des erfindungsgemäßen Systems;

**Fig. 3**   zeigt eine zweite exemplarische Ausführungsform des erfindungsgemäßen Systems; und Fresenius Medical Care Deutschland GmbH

**Fig. 4**   zeigt eine exemplarische Ausführungsform einer Basiseinheit eines erfindungsgemäßen Systems.

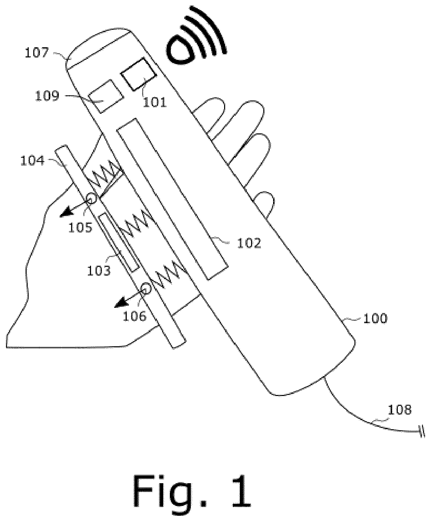

**Fig. 1** zeigt eine Ausführungsform des erfindungsgemäßen Elektrodenträgers 100. Dieser ist hier optional als Handelektrode oder Träger einer oder mehrerer Handelektroden ausgestaltet.

Der Elektrodenträger 100 verfügt über eine Funktionseinheit, die im Beispiel der Fig. 1 mindestens eine Radareinheit 101 aufweist.

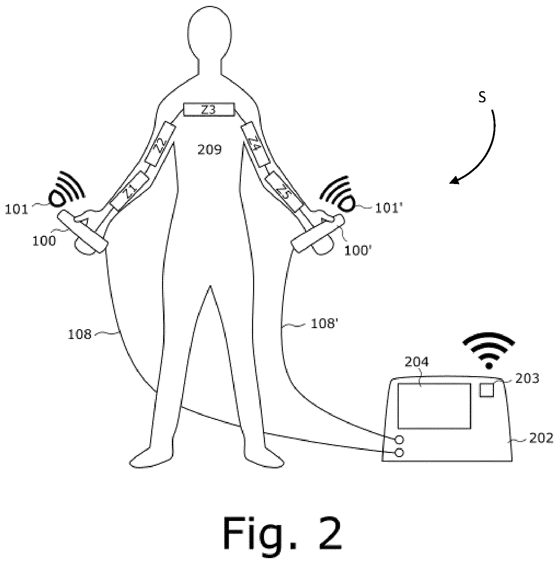

Die Radareinheit 101 ist dazu geeignet, Lagedaten mit anderen Vorrichtungen und/oder einer Basiseinheit 202 (siehe Fig. 2 bis Fig. 4) oder Daten, welche die Bestimmung der Lage des Elektrodenträgers 100 ermöglicht, auszutauschen. Letzteres kann die Messposition des Nutzers ermitteln helfen und hierüber dazu beitragen, die Genauigkeit und Verlässlichkeit der Impedanzmessung oder Impedanzanalyse zu erhöhen. Eine entsprechende Konfigurierung oder Programmierung der auswertenden Einheiten kann vorgesehen sein. Letzteres gilt für jeden der hierin genannten Verfahrensschritte.

Der Elektrodenträger 100 verfügt im Beispiel der Fig. 1 weiterhin über eine leitende Oberfläche 102, welche zur Durchführung einer Impedanzanalyse geeignet ist.

Beispielhaft ist zudem eine weitere oder zweite leitende Oberfläche 103 auf dem Elektrodenträger 100 vorgesehen.

Die weitere leitende Oberfläche 103 ist rein optional an oder auf einer Kraftmessvorrichtung 104, welche hier exemplarisch Fresenius Medical Care Deutschland GmbH ebenfalls Teil der Funktionseinheit ist, angebracht. Sie, oder das Zusammenspiel mit ihr, kann dazu dienen oder verwendet werden, den Einfluss der Hautoberfläche aus der Impedanzanalyse zu extrahieren, zu verringern oder gar eliminieren zu können.

Die Kraftmessvorrichtung 104 umfasst hier exemplarisch einen Sensor zum Bestimmen der Handkraft, welcher hier optional ebenfalls Teil der Funktionseinheit des erfindungsgemäßen Elektrodenträgers 100 ist.

Auf der Kraftmessvorrichtung 104 befinden sich - wiederum rein exemplarisch - zwei Grünlichtsensoren 105 und 106 als weitere Bestandteile der Funktionseinheit. Sie sind dazu geeignet, Vitalparameter wie beispielsweise den Herzschlag, Blutdruck und/oder die Sauerstoffsättigung des Nutzers zu erfassen. Eine entsprechende Ausgestaltung zum Auswerten und/oder Ausgeben von Auswertungsergebnissen vorstehende Parameter betreffend kann wiederum vorgesehen sein.

Die Kraftmessvorrichtung 104 kann einen Energiespeicher wie eine oder mehrere Federn oder dergleichen aufweisen, wie an dem in Fig. 1 gezeigten Beispiel gezeigt.

Weiterhin verfügt der Elektrodenträger 100 hier rein exemplarisch über einen Tastkopf 107, der dazu geeignet ist, durch Kombination einer weiteren Vorrichtung, beispielsweise einem weiteren Elektrodenträger, eine lokale Impedanzmessung vorzunehmen, um beispielsweise das Füllvolumen des Peritoneums oder der Bauchhöhle zu erfassen.

Der Tastkopf 107 ist, wie bereits die vorstehend genannten Komponenten, optionaler Teil der Funktionseinheit. Er kann Fresenius Medical Care Deutschland GmbH exemplarisch an einem Ende des/der Elektrodenträger(s) angeordnet sein. Alternativ oder ergänzend zum Tastkopf 107 kann eine Ultraschalltastsonde vorgesehen sein, die für sonografische Untersuchungen eingesetzt werden kann.

Der Elektrodenträger 100 ist zu seinem Verbinden mit einer Basiseinheit 202 (in Fig. 1 nicht gezeigt, siehe Fig. 2) mit einem Leiter 108 oder Kabel verbunden. Der Leiter 108 kann zur Stromversorgung und/oder Spannungsableitung der angeschlossenen Vorrichtungen und zum Datenaustausch dienen, z. B. zum Austausch von Daten der Kraftmessvorrichtung und/oder zum Austausch der Vitaldaten des Nutzers.

Alternativ oder ergänzend umfasst die Funktionseinheit eine optionale Anzeigevorrichtung 109. Die Anzeigevorrichtung 109 kann programmiert oder konfiguriert sein zum Anzeigen von Daten, die von ihr ermittelt wurden, oder von Daten, die ihr, beispielsweise von der Basiseinheit 202, hier nicht gezeigt, und/oder von anderer Stelle übermittelt wurden.

Die Anzeigevorrichtung 109 dient beispielsweise zur Kenntnisnahme der Daten durch den Nutzer und/oder Dritte.

**Fig. 2** zeigt eine exemplarische Ausführungsform eines erfindungsgemäßen Systems S bestehend aus zwei medizinischen Elektrodenträgern 100, 100', welche hier rein exemplarisch jeweils als Handelektroden ausgeführt sind, sowie einer Basiseinheit 202, oder diese Komponenten aufweisend.

Die Basiseinheit 202 verfügt optional über eine Kommunikationsvorrichtung 203, welche sich vorzugsweise, beispielsweise mittels WLAN, mit einem lokalen Netzwerk verbinden kann.

Fresenius Medical Care Deutschland GmbH

Ein berührungsempfindliches Display 204, oder Touchscreen, zur Steuerung des erfindungsgemäßen Systems S ist optional ebenfalls vorgesehen.

An die Basiseinheit 202 sind mittels Kabelverbindungen 108 bzw. 108' rein exemplarisch zwei Elektrodenträger 100, 100' angeschlossen.

Beide Elektrodenträger 100, 100' verfügen in dieser konkreten Ausführungsform jeweils über eine Radareinheit 101, 101', um miteinander und/oder mit der Basiseinheit 202 Lageinformationen auszutauschen. Durch Auswerten der so erhaltenen Lageinformationen durch das System oder Vorrichtungen hiervon kann beispielsweise festgestellt werden, ob der Nutzer während der Impedanzmessung die Arme ausgestreckt oder angewinkelt hat oder diese hängen lässt.

Durch den geschlossenen elektrischen Stromkreislauf zwischen Basiseinheit 202, den Vorrichtungen 100, 100' sowie beteiligten Muskelgruppen Z1 bis Z5 im Körper des Nutzers 209 kann eine Widerstandsmessung und bei deren Auswertung mittels Impedanzanalyse der Fluidzustand desselbigen bestimmt werden.

Durch die Abstandsinformationen, welche in Ausführungsformen wie dieser durch die Radareinheiten 101, 101' der Elektrodenträger 100, 100' bereitgestellt werden, kann eine erhöhte Genauigkeit bei der Auswertung erreicht werden.

Nicht gezeigt ist in Fig. 2, dass die Basiseinheit 202 mit einer optischen Vorrichtung oder einer Kamera ausgestattet sein kann, mittels welcher z. B. die Position der Fresenius Medical Care Deutschland GmbH Vorrichtungen 100, 100' zueinander, zum Körper oder im Raum bestimmt wird mit den hierin genannten Vorteilen.

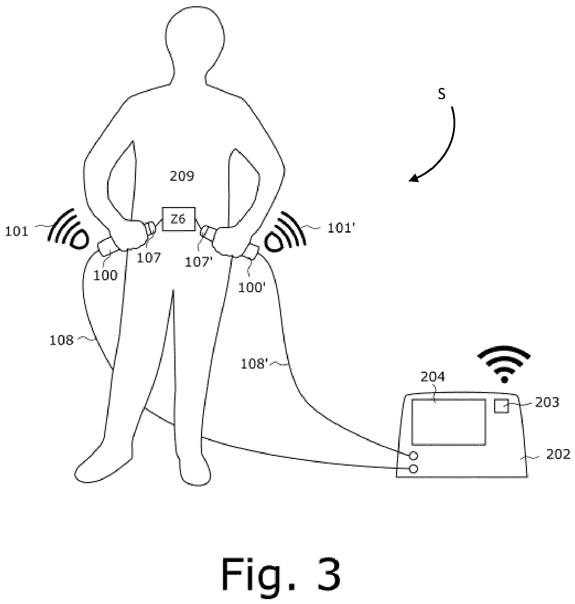

**Fig. 3** zeigt eine zweite exemplarische Ausführungsform des erfindungsgemäßen Systems S, bestehend aus bzw. umfassend zwei medizinische(n) Elektrodenträger(n) 100 und 100' sowie eine(r) Basiseinheit 202.

Es wird auf die Beschreibungen zur Fig. 2 Bezug genommen. Darin gezeigte, oder zu ihr genannte, Komponenten können optional auch Teil der zweiten Ausführungsform sein, und umgekehrt.

Die Basiseinheit 202, hier exemplarisch mit Kommunikationsvorrichtung 203 und/oder berührungsempfindlichem Display 204, ist in diesem Beispiel über die Kabelverbindungen 108 und 108' mit den beiden Elektrodenträgern 100 bzw. 100' verbunden.

Beide Elektrodenträger 100, 100' verfügen optional wiederum jeweils über je eine Radareinheit 101 und 101', die einen Datenaustausch und/oder eine Abstandmessung erlauben. Die Radareinheiten 101 und 101' sind bei dieser Ausführungsform besonders vorteilhaft, da wie gezeigt eine präzise Messung der Distanz zwischen beiden Elektrodenträgern 100, 100' ermöglicht wird, wobei sich die Messstrecke durch das Peritoneum bzw. den Peritonealraum, angedeutet durch das Bezugszeichen Z6, des Nutzers 209 erstreckt.

Weiterhin weisen die Elektrodenträger in diesem Beispiel jeweils einen optionalen Tastkopf 107 bzw. 107' auf, welche als Elektroden ausgestaltet sein können. Die Tastköpfe 107 Fresenius Medical Care Deutschland GmbH bzw. 107' erlauben, eine lokale Impedanz im Körper des Nutzers 209 zu ermitteln.

Wird die mittels der Tastköpfe 107, 107' ermittelte, insbesondere lokale, Impedanz in Relation zum mittels Radarsender/-empfänger ermittelten Abstand der Elektrodenträger 100, 100' gesetzt, kann dies in einer genaueren Impedanzanalyse resultieren.

Zum Vermessen des Peritonealraums stellen sich die Radareinheiten 101 und 101', insbesondere bei nichtplanen Oberflächen, als besonders geeignet heraus, wohingegen z. B. die Messung mittels Maßbandes, aufgrund der Körperkrümmung und dem Vorliegen von Bauchfett mit großen systematischen Fehlern einhergeht.

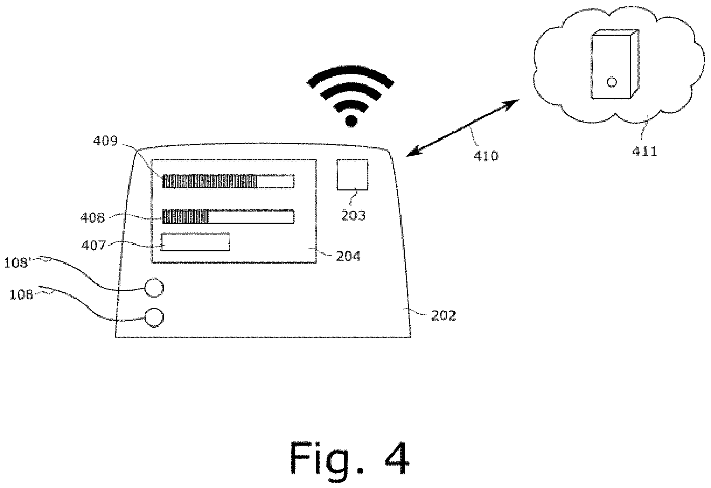

**Fig. 4** zeigt eine exemplarische Ausführungsform einer Basiseinheit 202 eines erfindungsgemäßen Systems S. In der Fig. 4 ist nur die Basiseinheit 202 in einer möglichen Ausführungsform gezeigt, für das restliche System wird auf die Ausführungen zu den Fig. 1 bis 3 verwiesen.

Die dargestellte Basiseinheit 202 verfügt über eine Kommunikationsvorrichtung 203, welche beispielsweise mittels WLAN und/oder Radar, Bluetooth, WLAN, 3G, LTE, 4G, 5G, ZigBee, NFC, Wibree und/oder WiMAX eine Verbindung mit einem externen Netzwerk und/oder mit einem oder mehreren Elektrodenträgern 100, 100' aufbauen kann.

Die Basiseinheit 202 ist weiterhin über zwei Leiter 108 und 108', hier: Kabelverbindungen, mit zwei erfindungsgemäßen Elektrodenträgern 100, 100' (in Fig. 4 nicht gezeigt) verbunden. Die Leiter 108 und 108' können der Stromversorgung Fresenius Medical Care Deutschland GmbH der Elektrodenträger 100, 100' und/oder zur Übermittlung von Daten, insbesondere von Spannungswerten, z. B. zur Impedanzmessung, dienen.

In bestimmten Ausführungsformen kann vorgesehen sein, auch weitere bzw. andere Daten, beispielsweise Vital- oder Fitnessdaten des Nutzers, auf diese Weise zwischen Elektrodenträgern 100, 100' und Basisstation 202 auszutauschen.

Auf dem Display 204 kann beispielsweise das Resultat der Kraftmessung der linken Hand 408 sowie der rechten Hand 409 jeweils unabhängig voneinander dargestellt werden.

Zusätzlich oder alternativ kann eine Nachricht 407 eingeblendet werden, die beispielsweise die Kraft des Nutzers in Relation zu vergleichbaren Nutzern ähnlichen Alters setzt, z. B. "Sie sind 78 % stärker als vergleichbare PD Patienten in ihrer Altersklasse".

Alternativ oder zusätzlich können auch Fitnessratschläge gegeben werden, z. B. "Wenn Sie täglich 15 Minuten mit Ihrer linken Hand trainieren, gleichen sich Fitnesslevel von linker und rechter Hand an".

Alternativ oder zusätzlich kann die vorliegende Erfindung auch verwendet werden, um die Handpumpaktionen von Patienten anzuregen bzw. zu überwachen, bei welchen eine neue arteriovenöse Fistel (Shunt) angelegt wurde. Die Handpumpaktionen dienen in diesen Fällen dazu, die beteiligte Vene zu maturieren, d. h. sie an die erhöhte Belastung bzw. den höheren Druck, der aufgrund der Fistel in ihr nun herrscht, anzupassen. Der erfindungsgemäße Elektrodenträger Fresenius Medical Care Deutschland GmbH eignet sich hierzu. Seine Funktionseinheit oder die Basiseinheit kann Trainingsvorgaben ausgeben und/oder deren Einhaltung überwachen, etwa basierend auf gespeicherten Trainingsprotokollen.

Die Daten können dabei über die Kommunikationsvorrichtung 203 der Basiseinheit 202 mit einem externen Rechner, Server und/oder einer Datencloud 411 ausgetauscht werden. Dabei können aktuelle Fitness- und/oder Gesundheitsdaten des Nutzers hochgeladen bzw. dessen historische Daten, deren Verlauf und/oder Vergleichsdaten anderer Patienten/Nutzer vom externen Rechner, Server und/oder der Datencloud 411 abgerufen werden, dies ist in Fig. 4 dargestellt mittels eines Doppelpfeils 410.

Die Daten der Kraftmessung können dabei lokal auf der Basiseinheit 202 und/oder über die Datencloud 411 auf einer entfernten Recheneinheit ausgewertet werden, um Rückschlüsse auf die Lebensqualität des Nutzers zu ziehen.

Ein kontinuierlicher Kraftverlust innerhalb einer kurzen Zeitspanne kann beispielsweise als Indiz hierfür dienen. In diesem Fall können auch weitere Nachrichten 407 angezeigt werden, die beispielsweise auf entsprechende Hilfsangebote für Patienten hinweisen.

Zusätzlich oder alternativ kann über die Kommunikationsvorrichtung 203 ein Kontakt zu einer medizinischen Fachkraft hergestellt werden, um den Nutzer bei Untersuchungen und/oder bei Impedanzmessungen mit dem beanspruchten System zu unterstützen.

**Bezugszeichenliste**

| | |
|---|---|
| 100 | medizinischer Elektrodenträger; hier exemplarisch mit oder als Handelektrode |
| 100' | medizinischer Elektrodenträger; hier exemplarisch mit oder als Handelektrode |
| 101 | Radareinheit als Beispiel einer Kommunikationsvorrichtung |
| 101' | Radareinheit als Beispiel einer Kommunikationsvorrichtung |
| 102 | leitende Oberfläche |
| 103 | weitere leitende Oberfläche |
| 104 | Kraftmessvorrichtung |
| 105 | Grünlichtsensor |
| 106 | Grünlichtsensor |
| 107 | Tastkopf; alternativ: Ultraschalltastkopf |
| 107' | Tastkopf; alternativ: Ultraschalltastkopf |
| 108 | Leiter; Kabel |
| 108' | Leiter; Kabel |
| 109 | Anzeigevorrichtung |
| | |
| 202 | Basiseinheit |
| 203 | Kommunikationsvorrichtung der Basiseinheit |
| 204 | Display; insbesondere berührungsempfindlich |
| 209 | Körper des Nutzers |
| | |
| 407 | Nachricht |
| 408 | Kraftmessung der linken Hand |
| 409 | Kraftmessung der rechten Hand |
| 410 | Fitness- und/oder Gesundheitsdatentransfer |
| 411 | Datencloud |
| | |
| S | System |
| Z1 bis Z5 | Muskelgruppen |
| Z6 | Peritonealraum oder Bauchhöhle (Cavitas peritonealis), vom Peritoneum umgeben |

**Patentansprüche**

1. Medizinischer Elektrodenträger (100, 100') mit

   - wenigstens einer leitenden Oberfläche (102) zu ihrem Einsatz als Elektrode in einer Impedanzmessung oder als Teil hiervon;
   - einer Funktionseinheit, welche wenigstens einen Sensor, einen Signalempfänger, einen Signalgeber, eine Kommunikationsvorrichtung, eine Auswerteeinheit, eine Kraftmessvorrichtung, ein Tastkopf (107, 107'), eine Trainingsvorrichtung, eine Kamera und/oder eine Anzeigevorrichtung (109), oder eine Kombinationen hiervon, ist oder aufweist; und

- einem Leiter (108, 108') zur elektrischen Verbindung der leitenden Oberfläche (102) mit einer Basiseinheit (202), welche dem Anlegen von elektrischer Spannung an die leitende Oberfläche (102) und/oder zum Auswerten von mittels, oder entlang, des Leiters (108, 108') übermittelten Spannungsinformationen zur Impedanzmessung oder mittels -analyse konfiguriert ist, oder mit einem Anschluss für einen solchen Leiter (108, 108').

2. Medizinischer Elektrodenträger (100, 100') nach Anspruch 1, wobei der medizinische Elektrodenträger (100, 100') oder die leitende Oberfläche (102) ausgestaltet sind als wenigstens eine Handelektrode, als wenigstens ein handgehaltenes Gerät und/oder wenigstens eine Fußelektrode, oder solche aufweist oder Teil hiervon sind.

3. Medizinischer Elektrodenträger (100, 100') nach einem der vorangehenden Ansprüche, wobei die Funktionseinheit als Kommunikationsvorrichtung zum Senden und/oder Empfangen von Daten ausgestaltet und/oder programmiert ist, oder eine solche aufweist, wobei die Funktionseinheit insbesondere als Kommunikationsvorrichtung, welche

- zum Senden von Daten an und/oder zum Empfangen von Daten von wenigstens einer weiteren Vorrichtung, wobei die weitere Vorrichtung ihrerseits vorzugsweise jeweils eine leitende Oberfläche (102) zu ihrem Einsatz als Elektrode in einer Impedanzmessung aufweist, ist oder umfasst oder Teil hiervon ist, und/oder als weiterer medizinischer Elektrodenträger (100, 100') ausgestaltet ist, und/oder
- zum Senden von Daten an und/oder zum Empfangen von Daten von einer Basiseinheit (202)

ausgestaltet und/oder programmiert ist, oder eine solche aufweist.

4. Medizinischer Elektrodenträger (100, 100') nach Anspruch 3, wobei die Kommunikationsvorrichtung als Radareinheit (101, 101'), z. B. mit Radarsender- und/oder Radarempfänger, ausgestaltet ist oder solche umfasst, wobei die übermittelten Daten vorzugweise Lagedaten oder Abstandsdaten sind oder umfassen.

5. Medizinischer Elektrodenträger (100, 100') nach einem der vorangehenden Ansprüche, wobei die Funktionseinheit eine Anzeigevorrichtung (109) aufweist und programmiert ist zum Anzeigen von von ihr ermittelten Daten oder ihr übermittelter Daten zur Kenntnisnahme durch einen Nutzer.

6. Medizinischer Elektrodenträger (100, 100') nach einem der vorangehenden Ansprüche, aufweisend wenigstens oder genau eine weitere, leitende Oberfläche, wobei wenigstens zwei leitende Oberflächen des medizinischen Elektrodenträgers (100, 100') getrennt voneinander und/oder an unterschiedlichen Positionen auf oder am medizinischen Elektrodenträger (100, 100') angeordnet sind.

7. Medizinischer Elektrodenträger (100, 100') nach einem der vorangehenden Ansprüche, wobei die Funktionseinheit konfiguriert ist, um basierend auf von ihr mittels Sensoren (104, 105, 106) erhobenen Daten ein Maß für die vom Nutzer empfundene Lebensqualität auszugeben.

8. Medizinischer Elektrodenträger (100, 100') nach einem der vorangehenden Ansprüche, wobei die Funktionseinheit eine Kraftmessvorrichtung (104) zum Bestimmen der Handkraft des Nutzers aufweist und/oder als Trainingsvorrichtung zum Trainieren der Handmuskulatur des Nutzers ausgestaltet und/oder programmiert ist.

9. Medizinischer Elektrodenträger (100, 100') nach einem der vorangehenden Ansprüche, wobei die Funktionseinheit wenigstens ein(en) Sensor (105, 106) zum Bestimmen eines oder mehrerer Vitalparameter des Nutzers ist oder aufweist und/oder wenigstens ein(en) Sensor (107, 107') zum Bestimmen der Hydrierung des Nutzers oder eines seiner Körperteile ist oder aufweist.

10. Medizinischer Elektrodenträger (100, 100') nach einem der vorangegangenen Ansprüche, wobei die Funktionseinheit ein Ultraschalltastkopf (107, 107') ist oder einen solchen aufweist.

11. System (S) zum Bestimmen der Körperzusammensetzung eines Nutzers mittels Impedanzanalyse, aufweisend:

- wenigstens einen medizinischen Elektrodenträger (100, 100') nach einem der Ansprüche 1 bis 10; und
- wenigstens eine Basiseinheit (202), zum Durchführen einer Impedanzanalyse;

wobei der medizinischen Elektrodenträger (100, 100') und die Basiseinheit (202) mittels eines Leiters (108, 108') zur Spannungsübertragung miteinander verbunden sind oder zur gemeinsamen Verbindung mittels des Leiters (108, 108') vorbereitet sind;

wobei die Basiseinheit (202) vorzugsweise wenigstens eine Schnittstelle zum Anschließen einer oder mehrerer Elektroden mit leitenden Oberflächen und/oder eines oder mehrerer medizinischen Elektrodenträger(s) (100, 100') aufweist.

**12.** System (S) nach Anspruch 11, wobei Basiseinheit (202) oder eine Recheneinrichtung des Systems (S), welche Teil der Basiseinheit (202) ist oder nicht ist, programmiert ist, um:

- mittels Impedanzanalyse einen Wert für die Muskelmasse oder für das Lean tissue des Nutzers zu berechnen;
- basierend sowohl auf dem mittels Impedanzanalyse berechneten Wert als auch auf der bestimmten Handkraft des Nutzers einen korrigierten Wert für die Muskelmasse oder für das Lean tissue des Nutzers zu berechnen.

**13.** System (S) nach einem der Ansprüche 11 bis 12, wobei das System ein berührungsempfindliches Display ist oder umfasst, konfiguriert oder programmiert zum Steuern oder Bedienen des Systems (S) oder der Basiseinheit (202) durch den Nutzer.

**14.** System (S) nach einem der Ansprüche 11 bis 13, wobei der medizinische Elektrodenträger (100, 100') und/oder die Basiseinheit (202) akustische, optische oder elektromagnetische und/oder gestengesteuerte, Kommunikationseinrichtungen zur Kommunikation mit dem Nutzer und/oder zu ihrer Bedienung durch den Nutzer, und/oder zur Kommunikation untereinander, aufweisen.

**15.** System (S) nach einem der Ansprüche 11 bis 14, wobei die Basiseinheit (202) zum Bestimmen der Hydrierung des Nutzers oder eines seiner Körperteile ausgestaltet und/oder programmiert ist, oder eine Vorrichtung hierfür aufweist und/oder wenigstens eine Auswerteeinheit zum Ausgeben einer Diagnose umfasst.

Fig. 1

Fig. 2

Fig. 3

Fig. 4

Europäisches
Patentamt

European
Patent Office

Office européen
des brevets

**EUROPÄISCHER RECHERCHENBERICHT**

Nummer der Anmeldung

EP 21 15 6357

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 111 481 199 A (HUAWEI TECH CO LTD) 4. August 2020 (2020-08-04) | 1-3,5,6, 8,9,11, 13-15 | INV. A61B5/0537 A61B5/22 A61B5/00 |
| A | * das ganze Dokument * | 4,10,12 | |
| X | JP 2000 023934 A (MATSUSHITA ELECTRIC IND CO LTD) 25. Januar 2000 (2000-01-25) | 1-3,5-7, 9,11, 13-15 | |
| A | * das ganze Dokument * | 4,8,10, 12 | |
| X | DE 20 2019 100532 U1 (GOLDENSUNDA TECH CO LTD [TW]) 19. Februar 2019 (2019-02-19) | 1-3,5-9, 11,13-15 | |
| Y | * Abbildungen 1-5,10 * | 12 | |
| A | | 4,10 | |
| X | US 2018/036531 A1 (SCHWARZ PHILIPP G [DE] ET AL) 8. Februar 2018 (2018-02-08) | 1-7, 9-11, 13-15 | |
| A | * Absätze [0015] - [0017], [0029], [0049], [0056], [0290], [0293], [0298], [0305], [0309]; Ansprüche 1,6; Abbildungen 1,5,14 * | 8,12 | |
| | | | RECHERCHIERTE SACHGEBIETE (IPC) |
| | | | A61B |
| X | US 2018/028810 A1 (SCHWARZ PHILIPP G [DE] ET AL) 1. Februar 2018 (2018-02-01) | 1,3,6,7, 9,11, 13-15 | |
| A | * Absätze [0021], [0030], [0035], [0036]; Abbildungen 1,10,11 * | 2,4,5,8, 10,12 | |
| X | US 2020/178873 A1 (PALO MATTI [US] ET AL) 11. Juni 2020 (2020-06-11) | 1,2,7,8, 11,13,14 | |
| Y | * Absatz [0043]; Abbildungen 1,9 * | 12 | |
| X | US 2012/172747 A1 (FUKUDA HIROAKI [IN] ET AL) 5. Juli 2012 (2012-07-05) * Abbildungen 2,6 * | 1,3,5-7, 11,13-15 | |

-/--

Der vorliegende Recherchenbericht wurde für alle Patentansprüche erstellt

| Recherchenort | Abschlußdatum der Recherche | Prüfer |
|---|---|---|
| Den Haag | 5. Juli 2021 | Lommel, André |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Seite 1 von 2

Europäisches Patentamt
European Patent Office
Office européen des brevets

## EUROPÄISCHER RECHERCHENBERICHT

**Nummer der Anmeldung**

EP 21 15 6357

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2007/129521 A1 (OMRON HEALTHCARE CO LTD [JP]; TOGOE YASUYUKI [JP] ET AL.) 15. November 2007 (2007-11-15) * Abbildungen 1-3,,7,10-15 * | 1,2,5-7, 11,14 | |
| Y | US 2018/168530 A1 (WEAR JAMES [US] ET AL) 21. Juni 2018 (2018-06-21) * Absatz [0026]; Abbildung 1 * | 12 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 5. Juli 2021 | Lommel, André |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 15 6357

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-07-2021

| Im Recherchenbericht angeführtes Patentdokument | Datum der Veröffentlichung | Mitglied(er) der Patentfamilie | Datum der Veröffentlichung |
|---|---|---|---|
| CN 111481199 A | 04-08-2020 | KEINE | |
| JP 2000023934 A | 25-01-2000 | JP 4069500 B2 | 02-04-2008 |
| | | JP 2000023934 A | 25-01-2000 |
| DE 202019100532 U1 | 19-02-2019 | CN 209916023 U | 10-01-2020 |
| | | DE 202019100532 U1 | 19-02-2019 |
| | | FR 3089292 A3 | 05-06-2020 |
| | | GB 2579421 A | 24-06-2020 |
| | | JP 3220932 U | 11-04-2019 |
| | | KR 20200001201 U | 09-06-2020 |
| | | TW M578150 U | 21-05-2019 |
| | | US 2020173873 A1 | 04-06-2020 |
| US 2018036531 A1 | 08-02-2018 | CN 107529995 A | 02-01-2018 |
| | | EP 3259016 A2 | 27-12-2017 |
| | | US 2018036531 A1 | 08-02-2018 |
| | | WO 2016131936 A2 | 25-08-2016 |
| US 2018028810 A1 | 01-02-2018 | CN 107530540 A | 02-01-2018 |
| | | EP 3259018 A1 | 27-12-2017 |
| | | US 2018028810 A1 | 01-02-2018 |
| | | WO 2016131935 A1 | 25-08-2016 |
| US 2020178873 A1 | 11-06-2020 | KEINE | |
| US 2012172747 A1 | 05-07-2012 | CN 102481109 A | 30-05-2012 |
| | | EP 2460466 A1 | 06-06-2012 |
| | | JP 4706782 B2 | 22-06-2011 |
| | | JP 2011024831 A | 10-02-2011 |
| | | US 2012172747 A1 | 05-07-2012 |
| | | WO 2011013345 A1 | 03-02-2011 |
| WO 2007129521 A1 | 15-11-2007 | JP 2007301093 A | 22-11-2007 |
| | | WO 2007129521 A1 | 15-11-2007 |
| US 2018168530 A1 | 21-06-2018 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5830137 A
- WO 2010022933 A1